(19) Europäisches Patentamt
European Patent Office
Office européen des brevets

(11) **EP 1 580 166 B1**

(12) **EUROPEAN PATENT SPECIFICATION**

(45) Date of publication and mention
of the grant of the patent:
**22.03.2017 Bulletin 2017/12**

(51) Int Cl.:
*C09C 1/36* (2006.01)    *A61K 8/29* (2006.01)
*A61Q 1/02* (2006.01)    *A61Q 1/06* (2006.01)

(21) Application number: **03774168.3**

(22) Date of filing: **21.11.2003**

(86) International application number:
**PCT/JP2003/014956**

(87) International publication number:
**WO 2004/052786 (24.06.2004 Gazette 2004/26)**

(54) **TITANIUM OXIDE PARTICLES HAVING USEFUL PROPERTIES**

TITANOXIDTEILCHEN MIT WERTVOLLEN EIGENSCHAFTEN

PARTICULES D'OXYDE DE TITANE AYANT DES PROPRIETES UTILES

(84) Designated Contracting States:
**AT BE BG CH CY CZ DE DK EE ES FI FR GB GR
HU IE IT LI LU MC NL PT RO SE SI SK TR**

(30) Priority: **09.12.2002 JP 2002356234**

(43) Date of publication of application:
**28.09.2005 Bulletin 2005/39**

(73) Proprietor: **Tayca Corporation
Osaka-shi,
Osaka-fu 551-0022 (JP)**

(72) Inventor: **ASAKURA, Takashi
Okayama-shi, Okayama 702-8006 (JP)**

(74) Representative: **Reitstötter Kinzebach
Patentanwälte
Sternwartstrasse 4
81679 München (DE)**

(56) References cited:
**EP-A- 0 870 731      WO-A1-97/24288
WO-A1-97/24289      JP-A- 10 316 429
JP-A- 11 011 948      JP-A- 59 199 530
JP-A- 62 275 182      JP-A- 63 225 532
US-A- 6 113 873**

**EP 1 580 166 B1**

**Description**

Field of the Invention

The present invention relates to titanium dioxide particles having beneficial properties such as highly selective shielding of thermal infrared radiation and highly spreadable property. Also disclosed herein is a method for producing such titanium dioxide particles.

Background of the Invention

Generally infrared radiation refers to electromagnetic radiation above the wavelength range of 0.76-0.83 $\mu$m of visible light reaching the wavelength of several millimeters. The solar radiation reaching the global surface comprises approximately 2 % of ultraviolet(UV), 48% of visible and 50% of infrared(IR) radiation. Most of IR are converted to thermal energy.

Titanium dioxide($TiO_2$) particles having a primary particle size range from about 0.2 $\mu$m to about 0.4 $\mu$m have a high refractive index and a high reflectivity to visible light, and thus have a high hiding power which makes the particles useful as white pigment for use in the production of paints, printing inks, plastic molding compounds, cosmetic preparations and so on.

$TiO_2$ microparticles having a primary particle size of less than 0.1 $\mu$m exhibit a low reflectivity and are transparent to visible light. However, they exhibit high shielding in the UV wavelength range that makes them useful as UV blocker in cosmetic and other preparations.

Because of their high reflectivity to visible light, the $TiO_2$ particles having a primary particle size from about 0.2 $\mu$m to about 0.4 $\mu$m used as white pigment are known to shield the visible wavelength range of the solar radiation. Therefore, they also have some heat shielding effect against the solar radiation. By "heat shielding effect" as used herein, it is meant the ability of preventing the elevation of internal temperature of an object exposed to the solar radiation by scattering the solar radiation on the surface thereof. In order to further increase the heat shielding effect, it would be required for $TiO_2$ particles to further increase the particle size. $TiO_2$ particles dedicated to shielding the thermal IR have not been developed to the best of our knowledge.

$TiO_2$ particles having different particle size range and optical properties from those of pigment grade and UV blocker $TiO_2$ particles are known. For example, JP-A-6018807 discloses a makeup cosmetic preparation comprising $TiO_2$ having a mean particle size in the range between 0.4 and 20 $\mu$m. This preparation is alleged to have aesthetically natural finish and improved extendability onto the skin. JP-A-09221411 discloses $TiO_2$ having a mean particle size greater than 0.10 $\mu$m and less than 0.14 $\mu$m. It is alleged that the $TiO_2$ in the above particle size has, when formulated in cosmetic preparations, a suitable level of hiding power while retaining UV blocking effect so as to impart aethetically natural finish free of pale appearance. JP-A-11158036 and JP-A-2000327518 disclose primary $TiO_2$ particles of 0.01 to 0.15 $\mu$m size that have been agglomerated into secondary particles of 0.6 to 2.0 $\mu$m size. They are formulated in cosmetic preparations in conjuction with plastic microbeads such as silione microbeads. The agglomerate is said to be transparent to visible light while retaining a large extent of the UV blocking effect of the primary particles without pale appearance. US Patent Number 6,113,873 describes specific anastase-type titanium dioxide compositions that are said to have high brightness and bluish white color.

$TiO_2$ of the white pigment grade has been adjusted to a primary particle size for efficiently scattering visible light in the wavelength range between 0.4 $\mu$m and 0.8 $\mu$m. Consequently, the ability thereof to shield thermal IR in the wavelength range higher than visible light is considered to be low in practice for IR shielding applications. If the IR shielding effect of $TiO_2$ can be increased significantly, it would find use as a thermal IR shielding material in a variety of compositions including paint compositions to be applied on houses and buildings, ships, automotives, household electrical and electronic equipment, drink cans, roads and the like to prevent them from exposing to an elevated temperature. Such a thermal IR shielding material would find use in cosmetic preparations for the prevention of elevated skin temperature.

In order to effectively shield thermal IR of the wavelength range between 0.8 $\mu$m and 3.0 $\mu$m, the $TiO_2$ particles need to have a wide distribution of the size of individual primary particles in the range between 0.4 $\mu$m and 1.5 $\mu$m in theory.

$TiO_2$ particles of pigment grade or UV blocker grade have been used in cosmetic preparations such as liquid foundations or powders as describe above. It is important for these preparations to have a high spreadability when applying to or spreading on the skin. The tactile feeling of cosmetic preparations containing $TiO_2$ of the pigment grade or UV blocker grade is not satisfactory due to $TiO_2$ particles themselves and the $TiO_2$ particles are often formulated in conjuction with a spreadability improver.

One approach for producing $TiO_2$ having larger particle size than the pigment grade $TiO_2$ in the existing plant for the sulfuric acid process would be to calcine hydrated $TiO_2$ at a temperature higher than the temperature at which hydrated $TiO_2$ is calcined to produce the pigment grade $TiO_2$. However, this process gives particles which are hardly dispersible in a medium as fine particles due to increased fraction of fused fine particles. Moreover, the growth of the particles in the direction of miner axis during the calcination is not sufficient compared to the direction of major axis resulting in generally rod-like particles having decreased scattering efficiency.

JP-B-50036440 discloses a process for producing a pigment grade $TiO_2$ comprising blending hydrated $TiO_2$ produced by the hydrolysis of titanyl sulfate with certain amounts of zinc sulfate and potassium sulfate, and calcining the blend at a temperature between 700°C and 1,000°C. The resulting $TiO_2$ contains a large amount of needle crystals of $TiO_2$, and the optical properties and primary particle size thereof are not different from the $TiO_2$ of pigment grade.

Brief Summary of the Invention

Accordingly, it is a principal object of the present invention to provide $TiO_2$ particles having a different primary particle size from that of the pigment grade or UV blocker grade and a number of beneficial properties including the ability of selectively shielding IR radiation and the ability of improving the spreadability of cosmetic preparations. Another object is to provide a process for producing the above $TiO_2$ particles.

The above and other objects of the present invention may be achieved by providing $TiO_2$ particles having a primary particle size between 0.5 and 2.0 $\mu$m and a reflectivity to the visible light less than 95%, obtainable by a process comprising:

blending hydrated $TiO_2$, based on the $TiO_2$ content thereof, with 0.1 to 0.5% by weight of an aluminum compound calculated as $Al_2O_3$, 0.2 to 1.0% by weight of zinc compound calculated as $ZnO$, and 0.1 to 0.5% by weight of a potassium compound calculated as $K_2CO_3$; and calcining the resulting blend at a temperature between 900°C and 1,100°C.

The $TiO_2$ particles thus produced contain at least 0.05 to 0.4% by weight of $Al_2O_3$ and 0.05 to 0.5% by weight of $ZnO$, the most part thereof, namely 0.05 to 0.3 wt.% of $Al_2O_3$ and 0.05 to 0.5 wt.% of $ZnO$ being present in the crystalline lattice.

The $TiO_2$ particles of the present invention may be incorporated into paints, printing inks or plastic molding compounds for shielding the thermal IR radiation. Due to relatively low reflectivity to the visible light, the $TiO_2$ particle of the present invention may be used in conjuction with conventional color pigments without whitening to impart a colored paint film with IR shielding effect. When incorporated in cosmetic preparations, the $TiO_2$ particles of the present invention may improve the spreadability in particular onto the skin compared to the pigment or UV blocker grade $TiO_2$. The $TiO_2$ particles of the present invention do not generate bluish luminescence observed in the $TiO_2$ white pigment due to decreased reflectivity to the visible light.

Brief Description of the Accompanying Drawings

Fig. 1 is a graph showing a transmission curve in the IR range of the film containing $TiO_2$ produced in Example 1.
Fig. 2 is a graph showing a transmission curve in the IR range of the film containing $TiO_2$ produced in Example 2.
Fig. 3 is a graph showing a transmission curve in the IR range of the film containing a commercial $TiO_2$ pigment.
Fig. 4 is a graph showing a transmission curve in the IR range of the film containing zinc titanate rather than $TiO_2$.
Fig. 5 is a graph showing a reflection curve in the visible range of $TiO_2$ produced in Example 1.
Fig. 6 is a graph showing a reflection curve in the visible range of $TiO_2$ produced in Example 2.
Fig. 7 is a graph showing a reflection curve in the visible range of a commercial $TiO_2$ pigment.

Detailed Description of the preferred Embodiments

According to the present invention, the $TiO_2$ particles having a primary particle size between 0.5 and 2.0 $\mu$m are produced starting from hydrated $TiO_2$. Blended with the hydrated $TiO_2$ are, based on the $TiO_2$ content thereof, an aluminum compound in an amount corresponding to 0.1 to 0.5% by weight calculated as $Al_2O_3$, a potassium compound in an amount corresponding to 0.1 to 0.5% by weight calculated as $K_2CO_3$, and a zinc compound in an amount corresponding to 0.2 to 1.0% by weight calculated as $ZnO$. After drying, the resulting blend is calcined at a temperature between 900°C and 1,100°C.

The starting hydrated $TiO_2$ may be produced, for example, by treating titanium-containing ore such as ilmenite or rutile with sulfuric or hydrochloric acid to remove impurities, and then adding water or an oxidizing agent to the resultant solution to precipitate hydrated $TiO_2$. Hydrated $TiO_2$ may be produced by hydrolyzing a titanium alkoxide. Metatitanic acid produced as an intermediate of $TiO_2$ pigment in the commercial sulfuric acid process is a preferred starting material.

Any aluminum compound may be added to hydrated $TiO_2$ provided that it does not adversely affects the desired properties of $TiO_2$ of the present invention. A water soluble aluminum salt such as the sulfate or chloride is preferred although the oxide or hydrated oxide may also be used. The amount of the aluminum compound to be added calculated as $Al_2O_3$ ranges between 0.1 and 0.5% by weight relative to the $TiO_2$ content of hydrated $TiO_2$.

Any potassium compound may also be used. Examples thereof include the hydroxide, carbonate or chloride. The amount of potassium compound to be added may range between 0.2 and 0.5% by weight calculated as $K_2CO_3$ relative to the $TiO_2$ content of hydrated $TiO_2$. In the absence or presence in only trace amounts of the potassium compound, a large portion of individual primary particles will be firmly fused together so that dispersion into individual primary particles will become difficult and the desired IR shielding effect will decrease. Conversely, excessive addition of the potassium compound will result in the formation of rod-like particles with decreased IR shielding effect or decreased conversion to rutile crystals in the desired particle size.

Any zinc compound may also be added to hydrated $TiO_2$. Preferred examples thereof include the oxide, sulfate or chloride. As disclosed herein, the amount of the zinc compound to be added ranges between 0.1 and 1.0% by weight calculated as $ZnO$ relative to the $TiO_2$ content of hydrated $TiO_2$. In the absence or presence in only trace amounts of the zinc compound, the proportion of rod-like particles with decreased IR shielding effect will increase.

Besides, dispersion of the product into individual primary particles will become difficult again due to firm fusion of primary particles together since a higher temperature is required for growing fine particles to the desired particle size in the presence of the zinc compound in excess. As is known in the art, the zinc compound reacts with $TiO_2$ at an elevated temperature to produce zinc titanate having a refractive index lower than $TiO_2$ pigment and hence the larger in the proportion of zinc titanate in the product the lower in the IR shielding effect. Excessive addition of the zinc compound is not preferable also for this reason.

The addition of aluminum, zinc and potassium compounds to hydrated $TiO_2$ may be achieved either by the dry process in which all components are physically bended in dry state or by the wet process in which an aqueous slurry of hydrated $TiO_2$ is used to uniformly disperse other components around each hydrated $TiO_2$ particle. Advantageously, the above components are added to a hydrated $TiO_2$ cake free from various impurities produced in a commercial $TiO_2$ pigment plant as an intermediate product, if necessary after dispersing the cake in an aqueous medium, and then the mixture is thoroughly stirred. The resulting mixture containing the aluminum, zinc and potassium additives is then de-hydrated to a hydrated $TiO_2$ content from 50 to 65% by weight prior to calcination at a temperature from 900 to 1100°C which is conventionally employed in the commercial $TiO_2$ pigment plant. When the calcination temperature is lower than the above range, the primary particles do not sufficiently grow to the desired size resulting in decreased IR shielding effect. Conversely, when the calcination temperature is higher than the above range, milling of the product into fine particles will become difficult due to excessive fusion or sintering also resulting in decreased IR shielding effect.

The $TiO_2$ particles of the present invention may optionally be coated with an amount of inorganic or organic coating materials sufficient to improve dispersibility, electrical property or weatherability necessary for incorporating to paint formulations or plastic molding compounds. Inorganic coating material may be those conventionally employed for coating $TiO_2$ pigments. Examples thereof are oxides or hydrated oxides of Al, Si, Zr, Zn, Ti, Sn, Sb or Ce. The oxide or hydrated oxide coating material may be formed in situ from, for example, sodium aluminate, aluminum sulfate, sodium silicate, hydrated silicic acid, zirconium sulfate, zirconium chloride, zinc sulfate, zinc chloride, titanyl sulfate, titanyl chloride, tin sulfate, tin chloride, antimony chloride, cerium chloride or cerium sulfate. Examples of organic materials includes aminosilanes, alkylsilanes, polyether silicone, silicone oil, stearic acid, magnesium stearate, zinc stearate, sodium stearate, lauric acid, alginic acid, sodium alginate, triethanolamine, or trimethylolpropane. The above coating material may be used in combination, and the species and amount thereof may be selected depending upon particular useage and desired properties.

The $TiO_2$ particles thus produced may be incorporated into paints, printing inks, plastic molding compounds or cosmetic preparations in order to impart with IR shielding effect.

For use in paint or printing ink formulations, the amount of $TiO_2$ particles of the present invention to be added may vary depending upon particular applications and generally ranges 1 to 500 weight parts per 100 weight parts of vehicle resin. Examples of the vehicle resins are acrylic-melamine, air-drying acrylic, acrylic-urethane, polyester-melamine, alkyd-melamine, polyurethane, nitrocellulose, fluoro, and vinyl chloride-vinyl acetate copolymer resins. The paint or printing ink formations may contain other pigments. Examples thereof include flaky pigments such as mica, sericite or talc, inorganic pigments such as calcium carbonate, barium sulfate, silica balloons, zirconium oxide, $TiO_2$ pigment, $TiO_2$ UV blocker, or zinc oxide, metal flakes such as aluminum flake, and inorganic or organic color pigments and dyes having a high transmission or reflectivity to IR wave range of the solar radiation. The $TiO_2$ particles of the present invention may be incorporated into paint or printing ink formulations as a suspension in water or organic solvent such as hydrocarbons, alcohols, ethers, esters, ester-alcohols or ketones. The mixture is then processed in a conventional apparatus such as paint conditioner, disper or sand grind mill to produce a uniform dispersion. The resulting formulation may be applied onto a metallic or plastic substrate using bar coater, brush, air spray gun or static coating machine to a desired film thickness. The coating film is then baked, depending upon the type of vehicle resin, at a temperature between 100°C and 180°C for a period of time between 10 minutes and 40 minutes.

For use in plastic molding compounds, the $TiO_2$ particles of the present invention are blended with a thermoplastic resin such as polyolefin, polystyrene, polyethylene terephthalate, or polyvinyl chloride. The amount of $TiO_2$ particles may vary depending upon particular applications of the product and generally ranges between 0.2 and 50 weight parts per 100 weight parts of the resin. The plastic molding compound may contain a lubricant, antioxidant or heat stabilizer. Examples thereof include zinc stearate, calcium stearate, aluminum stearate, magnesium stearate, zirconium stearate, calcium palmitate, sodium laurate and other fatty acid metal salts. These additives are preferably incorporated in an amount from 0.01 to 5% by weight of the plastic molding compound. The molding compound may optionally contain flaky pigment such as mica, sericite or talc, inorganic pigments such as calcium carbonate, barium sulfate, silica balloons, zirconium oxide, $TiO_2$ pigments, $TiO_2$ UV blocker, or zinc oxide, metal flakes such as aluminum flake, and inorganic or organic color pigments and dyes having a high transmission or reflectivity to IR wage range of the solar radiation. The $TiO_2$ particles may be blended with the resin by mixing them in a mixer such as tumbler or Henschel mixer in dry state and then kneading the mixture in molten state in Bunbury mixer, hot roll mill, extruder or injection molding machine.

Because the $TiO_2$ particles of the present invention have a primary particle size as large as from 0.5 to 2.0 $\mu$m, they have not only higher IR shielding effect but spreadability in comparison with known $TiO_2$ particles. The term "higher spreadability" as used herein refers to lower stationary and rolling friction coefficients against human skin. Therefore, the $TiO_2$ particles of the present invention may be added to foundational cosmetic preparations such as pressed powder foundation, powder foundation or liquid foundation, or makeup cosmetics such as face color, lip stick or rouge in order to improve spreadability. The $TiO_2$ particles may be incorporated in an amount of 1 to 50%. The cosmetic preparations or compositions may contain solid or semi-solid oil components such as vaseline, lanolin, sericin, microcrystalline wax, carnauba wax, candle wax, higher fatty acids or higher fatty alcohols, and/or fluid oil components such as squalane, paraffin oil, ester oil, diglyceride, triglyceride or silicone oil. Other components which are optionally added include hydrophilic or lipophilic polymers, surfactants, ethanol, preservatives, antioxidants, thickening agents, pH adjusting agents, perfumes, UV aborbers, moisturizers, blood circulation enhancers, frigidizers, astringents, disinfectants, and skin activators, Such components may be used to the extent of not adversely affecting the $TiO_2$ particles of the present invention. The cosmetic composition may contain conventional powder components. Examples thereof includes body pigments such as talc, kaoline, sericite, mica, magnesium carbonate, calcium carbonate, aluminum silicate, magnesium aluminosilicate, calcium silicate or anhydrous silicic acid; inorganic color pigments such as red iron oxide, black iron oxide, yellow iron oxide, ultramarine blue, prussian blue or carbon black; pearl pigments such as $TiO_2$-mica, iron oxide-mica or bismuth oxychloride; dyes such as tar or natural dyes; organic powders such as nylon powder, silicone powder, polyethylene or polypropylene powder, silk powder or crystalline cellulose, and inorganic UV blockers such as $TiO_2$ microparticles, ZnO microparticles or cerium oxide microparticles. Such powders may be surface-treated with fluorine compounds, silicone, metallic soap, wax, oil and fats, hydrocarbons or a combination thereof. The powder components may be used in conjuction with resins, oils, organic solvents, water or alcohols.

In order to incorporate the $TiO_2$ particles into cosmetic formulations, the particles may be surface-treated to improve their dispersibility in oily components or to give water-repellecy to the cosmetic formulation. The surface-treatment may be carried out by using known treating agents and known methods. Preferable treating agents are silicones such as methylhydrogenpolysiloxane and aluminum stearate. These agents may be used in combination with UV absorbers, surfactants or thickening agents. The treating method may be dry mixing in a mixer such as Henschel mixer or wet process in an organic solvent.

Example

The invention is further illustrated by the following Examples and Comparative Examples without limiting the invention thereto. All percentage and part are by weight unless otherwise indicated.

Example 1

$TiO_2$ particles having a primary particle size of 1.0 $\mu$m were produced by the following procedure.

A solution of titanyl sulfate was prepared by digesting ilmenite ore with hot concentrated sulfuric acid followed by removing impurities. The resulting solution was thermally hydrolyzed to obtain hydrated $TiO_2$ as a crude cake which was thoroughly washed with water to remove any electrolyte. To the purified cake was added an amount of a solution of aluminum sulfate corresponding to 0.2% calculated as $Al_2O_3$ relative to the $TiO_2$ content of the cake and the mixture was stirred for 15 minutes. To the mixture were added a solution of potassium hydroxide and a solution of zinc oxide successively in amounts 0.4% calculated as $K_2CO_3$ and 0.4% calculated as ZnO, respectively relative to the $TiO_2$ content of the cake followed by stirring for 15 minutes after each addition. Then the mixture was dried in a dryer at 110°C for 7 hours to obtain dry mixture having a $TiO_2$ content of about 60%. After drying, the mixture was calcined at 950°C for 2 hours. The calcined product was then pulverized in dry state in a sample mill and finely divided in wet state in a sand grinder mill to obtain an aqueous slurry having a $TiO_2$ content of about 24-29%. To the slurry was added an amount of sodium aluminate corresponding to 2.0% calculated as $Al_2O_3$ relative to the $TiO_2$ content followed by neutralization with sulfuric acid. Then the treated particles was collected by filtration, washed with water, and dried in a dryer at 110°C for 12 hours. Finally the dried product was divided into finer particles in a liquid energy mill.

A photograph of the resulting $TiO_2$ particle was taken using a transmission electron microscope (Jeol Ltd., model JEM-1230) and the volume average diameter was determined by measuring the diameter along the X-axis that divides the image of particles into equiareal halves (Martin's diameter) using an automated image analyzer (NIRECO, model LUZEX AP). The size of primary particles was about 1.0 $\mu$m.

Preparation of coating composition

A commercial clear acrylic lacquer and the above $TiO_2$ particle were weighed into a plastic mayonnaise bottle in 100 parts each as solids. After closing the bottle with a cap, the content was dispersed for 1 hour using a paint conditioner.

Preparation of test specimen

The above coating composition was applied onto a PET film to a dry film thickness of 5 $\mu$m using a automated bar coater, set for 10 minutes, and baked at 140°C for 30 minutes.

Example 2

The procedure of Example 1 for preparing $TiO_2$ particles, coating composition and test specimen was followed except that the calcination temperature was changed to 980°C. The primary particle size determined by the same way as in Example 1 was 1.2 $\mu$m.

Example 3

The procedure of Example 1 for preparing $TiO_2$ particles, coating composition and test specimen was followed except that the calcination temperature was changed to 1020°C. The primary particle size determined by the same way as in Example 1 was 1.5 $\mu$m.

Example 4

The procedure of Example 1 for preparing coating composition and test specimen was followed except that the amount of the same $TiO_2$ particle in the coating composition was changed to 50 parts.

Comparative Example 1

The procedure of Example 1 for preparing coating composition and test specimen was followed using a commercial $TiO_2$ pigment (Tayca Corporation, JR-701) instead of $TiO_2$ particles produced in Example 1. The primary particle size of the pigment determined by the same way as in Example 1 was 0.27 $\mu$m.

Comparative Example 2

The procedure of Example 1 for preparing coating composition and test specimen was followed by using zinc titanate particles instead of $TiO_2$ particle produced in Example 1.
The zinc titanate was produced as follows. The hydrated $TiO_2$ produced in Example 1 was mixed solely with an amount of zinc oxide corresponding to 200% calculated as ZnO relative to the $TiO_2$ content of hydrated $TiO_2$ oxide. The mixture was stirred for 15 minutes, dried to a $TiO_2$ content of 50-65% and calcined at 1000°C for 2 hours. After calcination, the product was processed in the same way as in Example 1. The calcined product was identified as zinc titanate using an X-ray diffractometer (Phillips, X'Pert Pro). The primary particle size of zinc titanate determined in the same way as in Example 1 was 1.0 $\mu$m.

Comparative Example 3

The procedure of Comparative Example 1 for preparing coating composition and test specimen was followed except that the amount of $TiO_2$ pigment in the coating composition was changed to 50 parts.

Comparative Example 4

The procedure of Comparative Example 1 for preparing coating composition and test specimen was followed except that the amount of TiO$_2$ pigment was changed to 40 parts.

Measurement of IR transmission

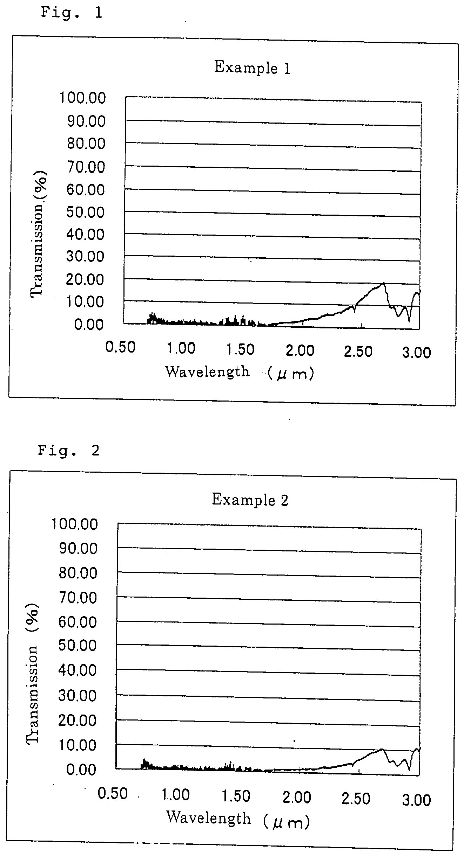

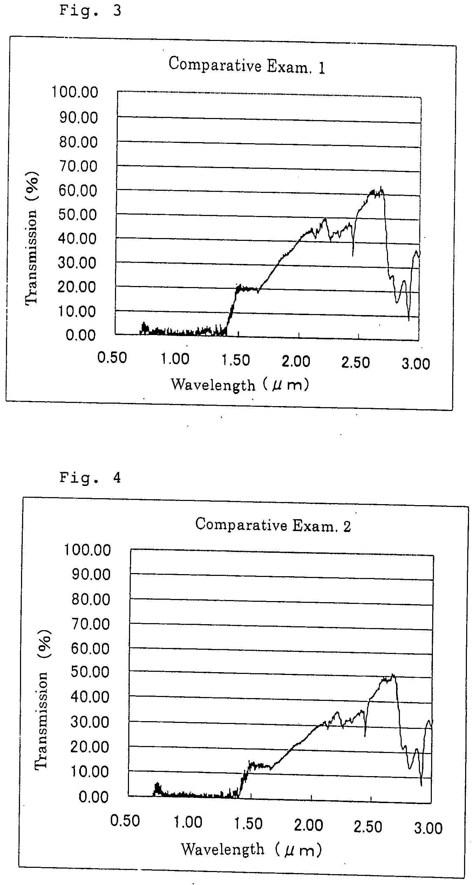

IR transmission was determined for test specimens containing TiO$_2$ at 50% level (Examples 1 and 2, Comparative Examples 1 and 2) using FT IR spectrophotometer (NIRECO, PRTGE 60) in the wavelength range from 0.7 to 3 $\mu$m. The transmission curve of each specimen is shown in Figs. 1-4.

The integrated IR transmission value over wavelength range between 1.4 and 3.0 $\mu$m was calculated from the curve of Figs. 1-4. Percent transmission is represented by the following equation and shown in Table 1 below.

$$\% \text{ transmission} = \frac{\text{integrated value of specimen}}{\text{integrated value of blank}} \times 100$$

Table 1

| Specimen | % IR transmission |
|---|---|
| Example 1 | 5 |
| Example 2 | 3 |
| Comp.Exm.1 | 36 |
| Comp.Exm.2 | 27 |
| PET film | 95 |

As shown in transmission curves of Figs. 1-4 and Table 1 above, the TiO$_2$ particles of the present invention exhibit remarkably lower IR transmission and higher shielding in the wavelength range from 1.4 to 3.0 $\mu$m compared to commercial TiO$_2$ pigment or zinc titanate particles.

Thermal IR shielding test

A small window of 40mm x 50mm size was cut on the top face of foamed polystyrene box and the window was closed with the film prepared in Examples 1-4 and Comparative Examples 1-4, respectively. The interior of the box was initially kept at room temperature (23°C). Then an IR lamp was placed at a distance of 15cm above the window and turned on for 20 minutes to irradiate the interior of the box with IR radiation. The inner temperature of the box was monitored each time and temperature differencial was determined at the end of irradiation by subtracting the inner temperature when irradiating through the film prepared in Examples and Comparative Examples from the inner temperature when irradiating through the control PET film not having any coating. The results are shown in Table 2 below.

Table 2

| Specimen | TiO$_2$ content | Inner Temp. | Temp.Diff. |
|---|---|---|---|
| Example 1 | 100 wt parts | 44°C | 30°C |
| Example 2 | " | 42°C | 32°C |
| Example 3 | " | 43°C | 31°C |
| Comp.Ex.1 | " | 51°C | 23°C |
| Comp.Ex.2 | " | 51°C | 23°C |
| Example 4 | 50 wt. parts | 48°C | 26°C |
| Comp.Ex.3 | " | 56°C | 18°C |
| Comp.Ex.4 | " | 58°C | 16°C |
| Control(PET) | None | 74°C | - |

As demonstrated in Table 2, the $TiO_2$ particles of the present invention retard elevation of temperature by shielding thermal IR radiation.

Example 5

Thermal IR shielding of $TiO_2$ particles of the present invention was evaluated in plastic molding compounds.

The $TiO_2$ particles produced in Example 2 were mixed with polyethylene in a proportion of 0.5 parts by weight relative to 100 parts by weight of polyethylene. The mixture was kneaded using a pair of hot rolls and pressed into a sheet having a thickness of 100 $\mu$m.

Comparative Example 5

Example 5 was followed using commercial $TiO_2$ pigment (Tayca, JR-701) instead of the $TiO_2$ particles of Example 2.

The thermal IR shielding test was repeated for the polyethylene sheets of Example 5 and Comparative Example 5 to determine temperature differential from the inner temperature of the box. The results are shown in Table 3 below. The temperature differential in Table 3 refers to the inner temperature differential between the sheet prepared in Example 5 or Comparative Example 5 and the corresponding polyethylene sheet to which $TiO_2$ was not added.

Table 3

| Specimen | $TiO_2$ content | Inner Temp. | Temp.Diff. |
|---|---|---|---|
| Example 5 | 0.5 wt.parts | 48°C | 7°C |
| Comp.Ex. 5 | 0.5 wt.parts | 51°C | 4°C |
| Control(PE sheet) | None | 55°C | - |

As demonstrated in Table 3, the $TiO_2$ particles of the present invention retard elevation of temperature by shielding thermal IR radiation when adding to plastic molding compounds.

Example 6

Thermal IR shielding of $TiO_2$ particles of the particles of the present invention was evaluated in cosmetic compositions.

Using the $TiO_2$ particles produced in Example 2, a cosmetic composition was prepared.

Formulation

| Powder components: | Wt. Parts |
|---|---|
| $TiO_2$ of Example 2 | 20 |
| Mica | 36 |
| Sericite | 10 |
| Talc | 10 |
| Oily components: | |
| Liquid paraffin | 17.5 |
| Isopropyl palmitate | 5 |
| Lipophilic glyceryl monooleate | 1.5 |

The powder components and oily components were separately mixed together. An antioxidant, preservative and perfume were dissolved q.v. in the oily component mixture. All components were placed in a ribbon blender and mixed well. The mixture was then compressed in a mold.

The resulting composition was applied uniformly on the entire surface of a surgical tape (8x5cm) using a finger in an amount of $2mg/cm^2$ to prepare a specimen.

Comparative Example 6

Example 6 was followed using commercial $TiO_2$ pigment (Tayca, JR-701) instead of the $TiO_2$ particles of Example 2 to produce the cosmetic composition and specimen.

The thermal IR shielding test as described above was repeated using the specimens prepared in Example 6 and Comparative Example 6. The results are shown in Table 4 below. The temperature differential therein refers to the inner temperature of the box closed with the specimen of Example 6 or Comparative Example 6 subtracted from the inner temperature of the box closed with untreated surgical tape.

Table 4

| Specimen | $TiO_2$ content | Inner Temp. | Temp.Diff. |
|---|---|---|---|
| Example 6 | 20 wt.parts | 35°C | 26°C |
| Comp.Ex.6 | 20 wt.parts | 40°C | 21°C |
| Control(surgical tape) | - | 61°C | - |

As demonstrated in Table 4, cosmetic compositions containing the $TiO_2$ particles of the present invention retard elevation of temperature by shielding thermal IR radiation.

Spreadability test of cosmetic compositions

Besides thermal IR shielding, the $TiO_2$ particles of the present invention can improve the spreadability of cosmetic compositions due to greater particle size than conventional $TiO_2$ pigment. The following are comparative experiments of the $TiO_2$ of the present invention and commercial $TiO_2$ pigment for spreadability.

Example 7

The $TiO_2$ particles produced in Example 1 having a primary particle size of 1.0 $\mu$m were surface-treated with dimethylpolysiloxane. Using the surface-treated $TiO_2$ particles, a pressed powder foundation composition was produced.

Formulation

| Powder components: | wt. parts |
|---|---|
| Surface-treated $TiO_2$ | 15 |
| Talc | 20 |
| Sericite | 30 |
| Mica | 20 |
| Iron oxide(red,yellow,black) | 3 |
| Oily components: | |
| Lanolin | 2.4 |
| Squalane | 2.4 |
| Capryloyl capryl triglyceride | 1.8 |
| 2-Ethylhexanoyl triglyceride | 1.8 |
| Methylphenylpolysiloxane | 3.6 |

The powder components were mixed in Henschel mixer for 5 minutes. To this were added portionwise the oily components heated to 50-60°C and mixing was continued for additional 5 minutes. The resulting mixture was transferred in a mold and compressed to obtain the desired product.

Example 8

Analoguous to Example 7, a pressed powder foundation composition was produced using the surface-treated $TiO_2$ particles of Example 1.

Formulation

| Powder components: | Wt. parts |
|---|---|
| Surface-treated $TiO_2$ | 10 |
| $TiO_2$ microparticles(Tayca,MT-100TV) | 1 |
| Talc | 19 |
| Sericite | 30 |

(continued)

| Powder components: | Wt. parts |
|---|---|
| Mica | 18 |
| Anhydrous silicic acid | 2.5 |
| Nylon powder | 4.5 |
| Iron oxide (red,yellow,black) | 3 |
| Oily components: | |
| Lanolin | 2.4 |
| Squalane | 2.4 |
| Capryloyl capryl triglyceride | 1.8 |
| 2-Ethylhexanoyl triglyceride | 1.8 |
| Methylphenylpolysiloxane | 3.6 |

The powder components were mixed in Henschel mixer for 5 minutes. To this were added portionwise the oily components heated to 50-60°C and mixing was continued for additional 5 minutes. The resulting mixture was transferred in a mold and compressed to obtain the desired product.

Comparative Example 7

The commercial $TiO_2$ pigment used in Comparative Example 1 was surface-treated with dimethylpolysiloxane. Using the surface-treated $TiO_2$ pigment, Example 7 was repeated to produce a pressed powder foundation composition.

Application feeling test:

The application feeling of cosmetic compositions such as liquid foundation and powders are affected by the spreadability of powder components incorporated therein.

The pressed foundations of Examples 7-8 and Comparative Example 7 were tested for the application feeling by applying the foundation directly to the skin of 10 test panelers. The application feeling sensed by the panelers was evaluated according to the following schedule.

Schedule

Very good: 8-10 panelers reported to be not creaky and highly spreadable.
Good: 6-7 panelers reported to be not creaky and highly spreadable.
Fair: 3-5 panelers reported to be not creaky and highly spreadable.
Bad: 0-2 panelers reported to be not creaky and highly spreadable.

The results are shown in Table 5 below.

| Composition | $TiO_2$ content | Application feeling |
|---|---|---|
| Example 7 | 15 wt.% | Very good |
| Example 8 | 10 wt.% | Very good |
| Comp.Ex.7 | 15 wt.% | Bad |

Reflectivity of Visible Light

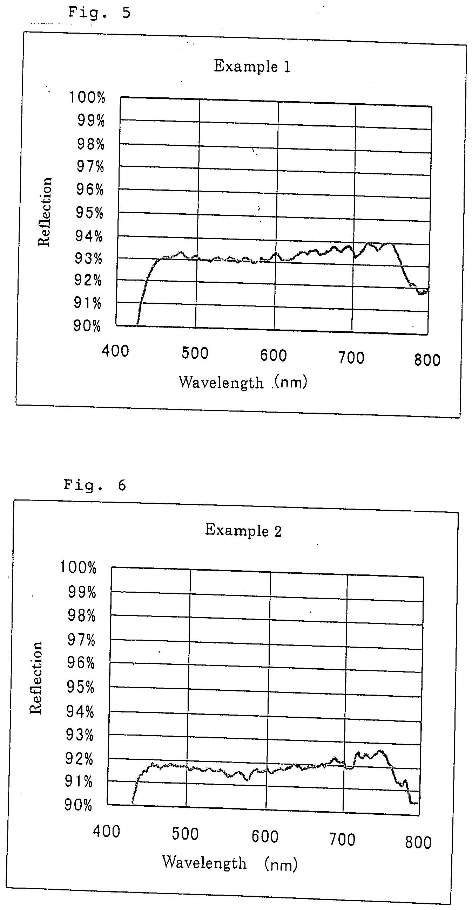

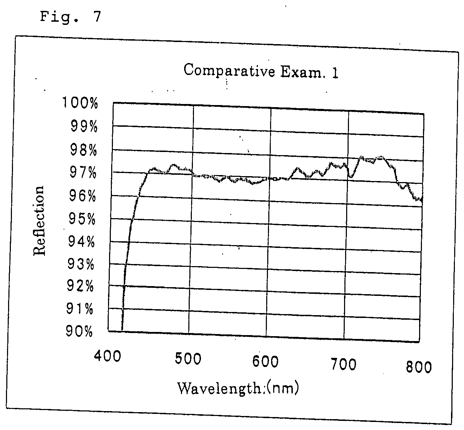

$TiO_2$ particles of Examples 1-2 and commercial $TiO_2$ pigment used in Comparative Example 1 were each compressed at 100 MPa into tablets. The reflectivity in the wavelength range between 0.4 and 0.8 $\mu$m relative to a standard MgO tablets was measured using a spectrophotometer (Hitachi Ltd., Model U-3000). Figs. 5-7 show the reflectivity curves of $TiO_2$ particles of Examples 1-2 and commercial $TiO_2$ pigment.

The percent reflection was calculated from integrated value of reflectivity curve of Figs. 5-7 according to the following equation.

$$\% \text{ reflection} = \frac{\text{integrated reflectivity value}}{\text{integrated value of standard}} \times 100$$

The results are shown in Table 6 below.

Table 6

| $TiO_2$ | % Reflection of visible light |
|---|---|
| Example 1 | 90.6 |
| Example 2 | 92.0 |
| Comp.Ex.1 | 96.4 |

$TiO_2$ particles having decreased visible light reflection are advantageous for use in cosmetic compositions because of less whitening and less blueish luminescent effects on the cosmetic composition than $TiO_2$ pigment.

**Claims**

1. Particulate titanium dioxide having a primary particle size between 0.5 and 2.0 $\mu$m and a reflectivity to visible light less than 95%, obtainable by a process comprising:

   blending hydrated titanium dioxide with 0.1 to 0.5 % by weight of an aluminum compound calculated as $Al_2O_3$, 0.2 to 1.0 % by weight of a zinc compound calculated as ZnO, and 0.1 to 0.5 % by weight of a potassium compound calculated as $K_2CO_3$, all percentage being based on the $TiO_2$ content of hydrated titanium dioxide; and calcining the blend at a temperature between 900°C and 1100°C.

2. The particulate titanium dioxide of claim 1 consisting essentially of 0.05 to 0.4 % by weight of aluminum oxide and 0.1 to 0.8 % by weight of zinc oxide, the balance being titanium oxide.

3. The particulate titanium oxide of claim 2 wherein 0.05 to 0.3 % by weight of aluminum oxide and 0.05 to 0.5 % by weight of zinc oxide are incorporated in the crystalline lattice.

4. The particulate titanium dioxide of claim 1 wherein in the preparation process said aluminum compound is selected from the group consisting of aluminum oxide, hydrated aluminum oxide, aluminum sulfate and aluminum chloride.

5. The particulate titanium dioxide of claim 1 wherein in the preparation process said zinc compound is selected from the group consisting of zinc oxide, zinc sulfate and zinc chloride.

6. The particulate titanium dioxide of claim 1 wherein in the preparation process said potassium compound is potassium hydroxide or potassium chloride.

7. The particulate titanium dioxide of claim 1 wherein the preparation process further comprises the steps of blending said hydrated titanium oxide as a wet cake before calcination with said aluminium, zinc and potassium compounds, and drying the wet cake so that the $TiO_2$ content is 50 to 60 % by weight of dried blend.

8. A paint or a printing ink comprising the particulate titanium oxide of any of claims 1 to 7.

9. A plastic molding compound comprising the particulate titanium dioxide of any of claims 1 to 7.

10. A cosmetic composition comprising the particulate titanium dioxide of any of claims 1 to 7.

11. Use of the particulate titanium dioxide of any of claims 1 to 7 in paints, printing inks or plastic molding compounds for shielding thermal IR radiation.

**12.** Use of the particulate titanium dioxide of any of claims 1 to 7 in a cosmetic composition for spreading on the skin.

**Patentansprüche**

1. Partikelförmiges Titandioxid mit einer Primärpartikelgröße zwischen 0,5 und 2,0 $\mu$m und einem Reflexionsvermögen für sichtbares Licht von weniger als 95 %, erhältlich durch ein Verfahren, bei dem man:

   hydratisiertes Titandioxid mit 0,1 bis 0,5 Gew.-% einer Aluminiumverbindung, gerechnet als $Al_2O_3$, 0,2 bis 1,0 Gew.-% einer Zinkverbindung, gerechnet als ZnO und 0,1 bis 0,5 Gew.-% einer Kaliumverbindung, gerechnet als $K_2CO_3$, mischt, wobei sich alle Prozentangaben auf den $TiO_2$-Gehalt von hydratisiertem Titandioxid beziehen; und
   die Mischung bei einer Temperatur zwischen 900 °C und 1100 °C calciniert.

2. Partikelförmiges Titandioxid nach Anspruch 1, im Wesentlichen bestehend aus 0,05 bis 0,4 Gew.-% Aluminiumoxid und 0,1 bis 0,8 Gew.-% Zinkoxid, wobei der Rest Titanoxid ist.

3. Partikelförmiges Titanoxid nach Anspruch 2, wobei 0,05 bis 0,3 Gew.-% Aluminiumoxid und 0,05 bis 0,5 Gew.-% Zinkoxid in das Kristallgitter inkorporiert sind.

4. Partikelförmiges Titandioxid nach Anspruch 1, wobei im Herstellungsverfahren die Aluminiumverbindung ausgewählt ist unter Aluminiumoxid, hydratisiertem Aluminiumoxid, Aluminiumsulfat und Aluminiumchlorid.

5. Partikelförmiges Titandioxid nach Anspruch 1, wobei im Herstellungsverfahren die Zinkverbindung ausgewählt ist unter Zinkoxid, Zinksulfat und Zinkchlorid.

6. Partikelförmiges Titandioxid nach Anspruch 1, wobei im Herstellungsverfahren die Kaliumverbindung Kaliumhydroxid oder Kaliumchlorid ist.

7. Partikelförmiges Titandioxid nach Anspruch 1, wobei das Herstellungsverfahren außerdem Schritte des Mischens des hydratisierten Titanoxids als feuchter Kuchen vor der Calcination mit den Aluminium-, Zink- und Kaliumverbindungen, und der Trocknung des feuchten Kuchens umfasst, so dass der $TiO_2$-Gehalt 50 bis 60 Gew.-% der getrockneten Mischung beträgt.

8. Anstrichmittel oder Druckertinte umfassend das partikelförmige Titanoxid nach einem der Ansprüche 1 bis 7.

9. Kunststoffmasse umfassend das partikelförmige Titandioxid nach einem der Ansprüche 1 bis 7.

10. Kosmetische Zusammensetzung umfassend das partikelförmige Titandioxid nach einem der Ansprüche 1 bis 7.

11. Verwendung des partikelförmigen Titandioxids nach einem der Ansprüche 1 bis 7 in Anstrichmitteln, Druckertinten oder Kunststoffmassen zum Abschirmen thermischer IR-Strahlung.

12. Verwendung des partikelförmigen Titandioxids nach einem der Ansprüche 1 bis 7 in einer kosmetischen Zusammensetzung zur Verteilung auf der Haut.

**Revendications**

1. Dioxyde de titane particulaire ayant une taille de particule primaire comprise entre 0,5 et 2,0 $\mu$m et une réflectivité de la lumière visible inférieure à 95 %, pouvant être obtenu par un procédé comprenant :

   le mélange de dioxyde de titane hydraté avec 0,1 à 0,5 % en poids d'un composé d'aluminium calculé en $Al_2O_3$, 0,2 à 1,0 % en poids d'un composé de zinc calculé en ZnO, et 0,1 à 0,5 % en poids d'un composé de potassium calculé en $K_2CO_3$, tous les pourcentages étant basés sur la teneur en $TiO_2$ de dioxyde de titane hydraté ; et la calcination du mélange à une température comprise entre 900 °C et 1100 °C.

2. Dioxyde de titane particulaire de la revendication 1 constitué essentiellement de 0,05 à 0,4 % en poids d'oxyde d'aluminium et 0,1 à 0,8 % en poids dioxyde de zinc, le complément étant de l'oxyde de titane.

3. Oxyde de titane particulaire de la revendication 2 dans lequel 0,05 à 0,3 % en poids d'oxyde d'aluminium et 0,05 à 0,5 % en poids d'oxyde de zinc sont incorporés dans le réseau cristallin.

4. Dioxyde de titane particulaire de la revendication 1 où, dans le procédé de préparation, ledit composé d'aluminium est choisi dans le groupe constitué de l'oxyde d'aluminium, l'oxyde d'aluminium hydraté, le sulfate d'aluminium et le chlorure d'aluminium.

5. Dioxyde de titane particulaire de la revendication 1 où, dans le procédé de préparation, ledit composé de zinc est choisi dans le groupe constitué de l'oxyde de zinc, le sulfate de zinc et le chlorure de zinc.

6. Dioxyde de titane particulaire de la revendication 1 où, dans le procédé de préparation, ledit composé de potassium est l'hydroxyde de potassium ou le chlorure de potassium.

7. Dioxyde de titane particulaire de la revendication 1, où le procédé de préparation comprend en outre les étapes de mélange dudit oxyde de titane hydraté sous la forme d'un gâteau humide avant calcination avec lesdits composés d'aluminium, de zinc et de potassium, et séchage du gâteau humide de sorte que la teneur en $TiO_2$ soit de 50 à 60 % en poids du mélange séché.

8. Peinture ou encre d'impression comprenant l'oxyde de titane particulaire de l'une quelconque des revendications 1 à 7.

9. Composé de moulage de matière plastique comprenant le dioxyde de titane particulaire de l'une quelconque des revendications 1 à 7.

10. Composition cosmétique comprenant le dioxyde de titane particulaire de l'une quelconque des revendications 1 à 7.

11. Utilisation du dioxyde de titane particulaire de l'une quelconque des revendications 1 à 7 dans des peintures, des encres d'impression ou des composés de moulage de matière plastique pour protéger contre un rayonnement IR thermique.

12. Utilisation du dioxyde de titane particulaire de l'une quelconque des revendications 1 à 7 dans une composition cosmétique pour étalement sur la peau.

Fig. 1

Example 1

Fig. 2

Example 2

Fig. 3

Comparative Exam. 1

Fig. 4

Comparative Exam. 2

Fig. 5

Example 1

Fig. 6

Example 2

Fig. 7

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP 6018807 A
- JP 09221411 A
- JP 11158036 A
- JP 2000327518 A
- US 6113873 A
- JP 50036440 B